(19)

Europäisches Patentamt

European Patent Office

Office européen des brevets

(11) **EP 4 198 991 A1**

(12) **EUROPEAN PATENT APPLICATION**
published in accordance with Art. 153(4) EPC

(43) Date of publication:
**21.06.2023 Bulletin 2023/25**

(21) Application number: **22766179.0**

(22) Date of filing: **28.02.2022**

(51) International Patent Classification (IPC):
***G16B 15/30*** (2019.01)

(52) Cooperative Patent Classification (CPC):
**G16C 20/50; G16B 15/30;** G16C 20/70

(86) International application number:
**PCT/CN2022/078336**

(87) International publication number:
**WO 2022/188653 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.03.2021 CN 202110260343**

(71) Applicants:
• **Tencent Technology (Shenzhen) Company Limited**
**Shenzhen, Guangdong 518057 (CN)**
• **Hitgen Inc.**
**Chengdu, Sichuan 610200 (CN)**

(72) Inventors:
• **XU, Tingyang**
**Shenzhen, Guangdong 518057 (CN)**

• **YU, Yang**
**Shenzhen, Guangdong 518057 (CN)**
• **RONG, Yu**
**Shenzhen, Guangdong 518057 (CN)**
• **LIU, Wei**
**Shenzhen, Guangdong 518057 (CN)**
• **HUANG, Junzhou**
**Shenzhen, Guangdong 518057 (CN)**
• **TU, Guiping**
**Chengdu, Sichuan 610200 (CN)**
• **QIU, Yaping**
**Chengdu, Sichuan 610200 (CN)**
• **CHENG, Xuemin**
**Chengdu, Sichuan 610200 (CN)**

(74) Representative: **Gunzelmann, Rainer**
**Wuesthoff & Wuesthoff**
**Patentanwälte PartG mbB**
**Schweigerstraße 2**
**81541 München (DE)**

(54) **MOLECULAR SCAFFOLD HOPPING PROCESSING METHOD AND APPARATUS, MEDIUM, ELECTRONIC DEVICE AND COMPUTER PROGRAM PRODUCT**

(57) A method and an apparatus for processing molecular scaffold transitions, a computer-readable medium, an electronic device, and a computer program product are provided. The method for processing molecular scaffold transitions includes: generating, according to a connection graph structure corresponding to a reference drug molecule, an atomic latent vector corresponding to the reference drug molecule; performing atom masking processing on the atomic latent vector to obtain a scaffold latent vector and a sidechain latent vector included in the atomic latent vector; generating a target scaffold latent vector with a target transition degree between the scaffold latent vector and the target scaffold latent vector according to a spatial distribution of the scaffold latent vector; and generating a transitioned drug molecule according to the target scaffold latent vector and the sidechain latent vector.

| Generate, according to a connection graph structure corresponding to a reference drug molecule, an atomic latent vector corresponding to the reference drug molecule | S210 |
| Perform atom masking processing on the atomic latent vector corresponding to the reference drug molecule to obtain the scaffold latent vector and the sidechain latent vector included in the atomic latent vector | S220 |
| Generate a target scaffold latent vector with a target transition degree between the scaffold latent vector and the target scaffold latent vector according to a spatial distribution of the scaffold latent vector | S230 |
| Generate a transitioned drug molecule according to the target scaffold latent vector and the sidechain latent vector | S240 |

FIG. 2

EP 4 198 991 A1

**Description**

CROSS-REFERENCE TO RELATED APPLICATION

Embodiments of this application are based on and claim priority to Chinese Patent Application No. 202110260343.1 filed on March 10, 2021, which is incorporated herein by reference in its entirety.

FIELD OF THE TECHNOLOGY

This application relates to the field of computer and communication technologies, and specifically, to a method and an apparatus for processing molecular scaffold transition, a medium, an electronic device, and a computer program product.

BACKGROUND OF THE DISCLOSURE

Scaffold transition is a very important tool for pharmacochemical design. Its main purpose is to change an existing molecular structure, replace a local structure of a complex natural product, and/or improve a pharmacokinetic property of a molecule by changing a scaffold of the molecule.

Scaffold transition solutions in the related art are mainly based on methods such as the pharmacophore model, molecular shape similarity searches, and other schemes. However, because these solutions are all generated based on rules, it is difficult to get rid of design ideas of pharmacochemical experts, resulting in lack of novelty of a transitioned molecule.

SUMMARY

Embodiments of this application provide a method and an apparatus for processing a molecular scaffold transition, a medium, and an electronic device, thereby improving novelty of a newly generated drug molecule.

Other features and advantages of this application become obvious through the following detailed descriptions, or may be partially learned partially through the practice of this application.

Some embodiments of this application provide a method for processing molecular scaffold transitions. The method includes generating, according to a connection graph structure corresponding to a reference drug molecule, an atomic latent vector corresponding to the reference drug molecule. The method includes performing atom masking processing on the atomic latent vector to obtain a scaffold latent vector and a sidechain latent vector included in the atomic latent vector. The method includes generating a target scaffold latent vector with a target transition degree between the scaffold latent vector and the target scaffold latent vector according to a spatial distribution of the scaffold latent vector. The method includes generating a transitioned drug molecule according to the target scaffold latent vector and the sidechain latent vector.

Some embodiments of this application provide an apparatus for processing a molecular scaffold transition. The apparatus includes: a first generation unit, configured to generate, according to a connection graph structure corresponding to a reference drug molecule, an atomic latent vector corresponding to the reference drug molecule; a first processing unit, configured to perform atom masking processing on the atomic latent vector to obtain a scaffold latent vector and a sidechain latent vector included in the atomic latent vector; a second generation unit, configured to generate a target scaffold latent vector with a target transition degree between the scaffold latent vector and the target scaffold latent vector according to a spatial distribution of the scaffold latent vector; and a third generation unit, configured to generate a transitioned drug molecule according to the target scaffold latent vector and the sidechain latent vector.

Some embodiments of this application further provide a computer-readable medium, storing a computer program. The computer program, when executed by a processor, causes the method for processing a molecular scaffold transition according to the foregoing embodiments to be implemented.

Some embodiments of this application provide an electronic device, including: one or more processors; a storage apparatus, configured to store one or more programs. The one or more programs, when executed by the one or more processors, cause the one or more processors to implement the method for processing molecular scaffold transitions according to the foregoing embodiments.

An embodiment of this application provides a computer program product or a computer program. The computer program product or the computer program includes a computer instruction. The computer instruction is stored in a computer-readable storage medium. A processor of a computer device reads the computer instructions from the computer-readable storage medium. The processor executes the computer instructions, to cause the computer device to perform the method for processing a molecular scaffold transition shown in the foregoing various exemplary embodiments.

In the technical solutions provided in some embodiments of this application, the atom masking processing is performed on the atomic latent vector corresponding to the drug molecule to obtain the scaffold latent vector and the sidechain latent vector. Then, according to the spatial distribution of the scaffold latent vector, the target scaffold latent vector having the target transition degree is generated, so that the transitioned drug molecule is generated according to the target scaffold latent vector and the sidechain latent vector. Therefore, by mapping the scaffold latent vector to the spatial distribution, the generated target scaffold latent vector can get rid of a design mindset of pharmaceutical experts, and good novelty can be achieved. In addition, the solution can be automatically executed through the electronic device to improve efficiency of the scaffold transition.

It is to be understood that the foregoing general descriptions and the following detailed descriptions are merely for illustration and explanation purposes and are not intended to limit this application.

BRIEF DESCRIPTION OF THE DRAWINGS

FIG. 1 is a schematic diagram of an exemplary system architecture to which a technical solution according to an embodiment of this application is applicable.

FIG. 2 is a flowchart of a method for processing a molecular scaffold transition according to an embodiment of this application.

FIG. 3A is a flowchart of generating an atomic latent vector corresponding to a reference drug molecule according to an embodiment of this application.

FIG. 3B is a flowchart of performing atom masking processing on an atomic latent vector corresponding to a reference drug molecule according to an embodiment of this application.

FIG. 3C is a flowchart of generating a target scaffold latent vector having a specified transition degree according to an embodiment of this application.

FIG. 4A is a schematic structural diagram of a machine learning model according to an embodiment of this application.

FIG. 4B is a schematic diagram of a processing process of a graph encoder according to an embodiment of this application.

FIG. 5 is a schematic diagram of atom masking and graph readout part according to an embodiment of this application.

FIG. 6 is a schematic diagram of a distance representing method according to an embodiment of this application.

FIG. 7 is a schematic diagram of a processing process of a decoder according to an embodiment of this application.

FIG. 8 is a schematic diagram of a processing process of generating a scaffold latent vector and a sidechain latent vector through a model according to an embodiment of this application.

FIG. 9 is a schematic diagram of a decoding process through a model according to an embodiment of this application.

FIG. 10 is a schematic diagram of a scaffold transition method according to an embodiment of this application.

FIG. 11 is a block diagram of an apparatus for processing a molecular scaffold transition according to an embodiment of this application.

FIG. 12 is a schematic structural diagram of a computer system adapted to implement an electronic device according to an embodiment of this application.

DESCRIPTION OF EMBODIMENTS

Exemplary implementations are now described more comprehensively with reference to the accompanying drawings. However, the examples of implementations may be implemented in multiple forms, and it is not to be understood as being limited to the examples of implementations described herein. Conversely, the implementations are provided to make this application more comprehensive and complete, and comprehensively convey the idea of the examples of the implementations to a person skilled in the art.

In addition, the described features, structures, or characteristics may be combined in one or more embodiments in any appropriate manner. In the following descriptions, more specific details are provided to provide a comprehensive understanding of the embodiments of this application. However, a person skilled in the art is to be aware that, the technical solutions in this application may be implemented without one or more of the specific details, or another method, unit, apparatus, or step may be used. In other cases, well-known methods, apparatuses, implementations, or operations are not shown or described in detail, to avoid obscuring aspects of this application.

The block diagrams shown in the accompanying drawings are merely functional entities and do not necessarily correspond to physically independent entities. That is, the functional entities may be implemented in a software form, or in one or more hardware modules or integrated circuits, or in different networks and/or processor apparatuses and/or microcontroller apparatuses.

The flowcharts shown in the accompanying drawings are merely examples for descriptions, do not need to include all content and operations/steps, and do not need to be performed in the described orders either. For example, some operations/steps may be further divided, while some operations/steps may be combined or partially combined. Therefore, an actual execution order may change according to an actual case.

"Plurality of" mentioned in the specification means two or more. The "and/or" describes an association relationship for describing associated objects and represents that three relationships may exist. For example, A and/or B may represent the following three cases: Only A exists, both A and B exist, and only B exists. The character "/" generally indicates an "or" relationship between the associated objects.

The solutions provided in the embodiments of this application relate to technologies such as machine learning of artificial intelligence, and in particular, to applying the machine learning technology to a solution of scaffold transition of a drug molecule.

Before the solution of the molecular scaffold transition according to the embodiments of this application is introduced, the processing solutions in the related art is introduced first. The scaffold transition solution provided in the related art is mainly based on a pharmacophore model, molecular shape-based search, search based on chemical similarity of fingerprint, and an algorithm of machine learning.

Among them, the pharmacophore model simulates an active conformation of a ligand molecule through conformation search and molecular superposition, that is, retaining a molecular framework of a feature atom necessary for activity. The biggest feature of the pharmacophore is that it has a group of molecular interaction features shared by active molecules. In other words, the pharmacophore does not represent a real molecule or a group of chemical groups. Rather, it is an abstract concept. Features of the pharmacophore include: an acceptor and a donor of a hydrogen bond, an interaction between positive and negative charges, a hydrophobic interaction, an aromatic ring interaction, and the like. If such a pharmacophore feature can migrate from one molecule to another. That is, if a reference molecule and a test molecule have the same pharmacophore feature, a scaffold transition can be achieved. A similar solution is a drug design method based on a protein structure, in which an interaction between a small molecule and a residue of a binding site in a protein is expressed as a vector, and then a corresponding molecule having the same feature vector is searched for in a compound library, so as to achieve the scaffold transition.

The molecular shape-based search is mainly a search in which a volume in a molecular space is considered to search for similarity, binding with a target protein is expected to be maintained, and a scaffold replacement is achieved. A problem of this solution and other search solutions is that search time is very long, and limited by the existing chemical space, the search can only be performed in the existing compound library. In addition, there are many false positive molecules, which makes it difficult to ensure activity of the molecules.

With the development of artificial intelligence technology, especially its application in the generation of molecules, the ability of drug development has been accelerated. The biggest advantage of the molecular generation is that a brand-new molecule is generated, which directly realizes the de novo design of a drug molecule and expands the existing molecular space. AI algorithm-based molecular generation methods provided in the related art pay too much attention to a reconstruction capability and legitimacy of a molecule and render it difficult to meet the actual requirements of a pharmaceutical company. For example, a pharmaceutical company prefers to modify the existing molecule and keep its activity while getting rid of the existing structure. However, although the solutions in the related art can meet a requirement in activity maintenance, it is difficult to get rid of design ideas of pharmaceutical experts because the solutions are all based on rules, resulting in lack of novelty of a newly-generated molecule.

Based on the foregoing problems, the embodiments of this application provide a novel processing solution for molecular scaffold transition, through which a scaffold latent vector can be mapped to a spatial distribution, so that a generated target scaffold latent vector can get rid of a design mindset of pharmaceutical experts, and good novelty can be achieved. In addition, the solution can be automatically executed through an electronic device, which reduces manpower and time costs. The technical solutions of the embodiments of this application are described in detail in the following.

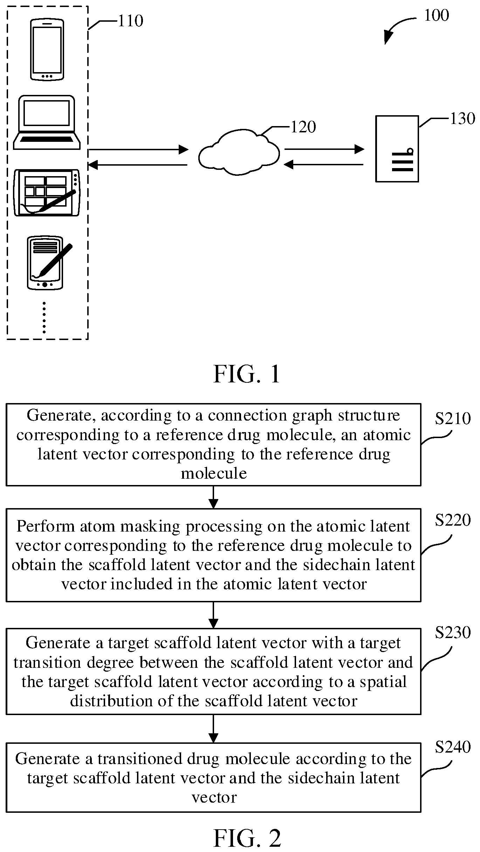

FIG. 1 is a schematic diagram of an exemplary system architecture to which a technical solution according to an embodiment of this application is applicable.

As shown in FIG. 1, a system architecture 100 may include a terminal 110, a network 120, and a server 130. The terminal 110 and the server 130 are connected through the network 120.

In the embodiments of this application, the terminal 110 may be a smartphone, a tablet computer, a notebook computer, a desktop computer, a smart speaker, a smart watch, and the like, but is not limited thereto. The network 120 may be a communication medium of various connection types capable of providing a communication link between the terminal 110 and the server 130, for example, a wired communication link, a wireless communication link, a fiber-optic cable, or the like. This is not limited in the embodiments of this application. The server 130 may be an independent physical server, or may be a server cluster or a distributed system formed by a plurality of physical servers, or may be a cloud server that provides basic cloud computing services such as a cloud service, a cloud database, cloud computing, a cloud function, cloud storage, a network service, cloud communication, a middleware service, a domain name service, a security service, a content delivery network (CDN), big data, and an artificial intelligence platform.

It is to be understood that the number of the terminals 110, networks 120, and the servers 130 in FIG. 1 are merely illustrative. There may be any number of terminals 110, any number of networks 120, and any number of servers 130 according to an implementation requirement. For example, the server 130 may be a server cluster including a plurality of servers.

In the embodiments of this application, a user may submit a reference drug molecule, that is, a molecule on which scaffold transition processing is required to be performed, to the server 130 by using the terminal 110 through the network 120, and may identify a scaffold required to be transitioned. Identifying the scaffold required to be transitioned is not a necessary process. After obtaining the reference drug molecule, the server 130 may convert a structure of the reference drug molecule into a connection graph structure, and then generate, according to the connection graph structure corresponding to the reference drug molecule, an atomic latent vector corresponding to the reference drug molecule.

After generating the atomic latent vector corresponding to the reference drug molecule, the server 130 may perform atom masking processing on the atomic latent vector to obtain a scaffold latent vector and a sidechain latent vector included in the atomic latent vector. In order to implement scaffold transition processing, a target scaffold latent vector having a target transition degree between the scaffold latent vector and the target scaffold latent vector is generated according to a spatial distribution of the scaffold latent vector, and then a transitioned drug molecule is generated according to the target scaffold latent vector and the sidechain latent vector obtained above.

By using the technical solutions in the embodiments of this application, a scaffold latent vector can be mapped to a spatial distribution, so that a generated target scaffold latent vector can get rid of a design mindset of pharmaceutical experts, and good novelty can be achieved. In addition, the solution can be automatically executed through a device, which reduces manpower and time costs.

The method for processing a molecular scaffold transition provided in the embodiments of this application is generally executed by the server 130, and accordingly, the apparatus for processing a molecular scaffold transition is generally disposed in the server 130. However, in another embodiment of this application, the terminal device may also have functions similar to those of the server, so as to execute the solution for processing a molecular scaffold transition provided in the embodiments of this application.

In the embodiments of this application, a user may submit a reference drug molecule through the terminal 110, that is, a molecule on which scaffold transition processing is required to be performed, and may identify a scaffold required to be transitioned. Identifying the scaffold required to be transitioned is not a necessary process. After obtaining the reference drug molecule, the terminal 110 can convert a structure of the reference drug molecule into a connection graph structure, and then generate, according to the connection graph structure corresponding to the reference drug molecule, an atomic latent vector corresponding to the reference drug molecule.

After generating the atomic latent vector corresponding to the reference drug molecule, the terminal 110 may perform atom masking processing on the atomic latent vector to obtain a scaffold latent vector and a sidechain latent vector included in the atomic latent vector. In order to implement scaffold transition processing, a target scaffold latent vector having a target transition degree between the scaffold latent vector and the target scaffold latent vector is generated according to a spatial distribution of the scaffold latent vector, and then a transitioned drug molecule is generated according to the target scaffold latent vector and the sidechain latent vector obtained above.

Implementation details of the technical solutions of the embodiments of this application are described below in detail.

FIG. 2 is a flowchart of a method for processing a molecular scaffold transition according to an embodiment of this application. The method for processing a molecular scaffold transition may be executed by an electronic device having a calculation processing function, for example, the server 130 shown in FIG. 1. Referring to FIG. 2, the method for processing a molecular scaffold transition includes at least steps S210 and S240. A detailed description is as follows:

Step S210. Generate, according to a connection graph structure corresponding to a reference drug molecule, an atomic latent vector corresponding to the reference drug molecule.

In the embodiments of this application, the reference drug molecule is a molecule requiring scaffold transition processing, and the connection graph structure corresponding to the reference drug molecule is a connection graph structure obtained according to structure conversion of the reference drug molecule.

For example, the connection graph structure corresponding to a drug molecule can be represented as $G = (A, X, E)$. $A$ represents a connection matrix, $X$ represents a node feature, and $E$ represents a side feature. In the connection graph structure, a node represents an atom in the drug molecule. The node feature is used for representing an atomic feature in the drug molecule, which may include: atomic mass, atomic charge number, atomic type, valence state, whether an atom is in a ring, whether it is an atom in an aromatic ring, and the like. The side feature is used for representing a feature between atoms in the drug molecule, which may include: whether a side is single bond or double bond, whether the side is in the ring, whether the side is in the aromatic ring, and the like.

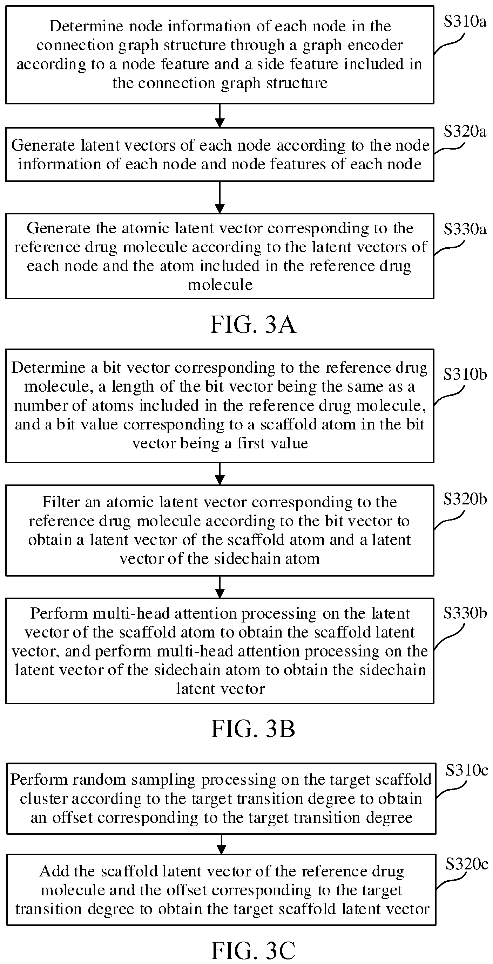

In the embodiments of this application, after the connection graph structure corresponding to the reference drug molecule is generated, the atomic latent vector corresponding to the reference drug molecule can be generated according to the connection graph structure corresponding to the reference drug molecule, which is specifically shown in FIG. 3A, and includes the following step S310a, step S320a, and step S330a. The detail is described as follows:

Step S310a. Determine node information of each node in the connection graph structure through a graph encoder according to a node feature and a side feature included in the connection graph structure.

In the embodiments of this application, for ease of description, a node $v$ and a node $w$ in the connection graph structure are used as an example, assuming that a node feature of the node $v$ can be represented as $x_v$, a node feature of the node $w$ can be represented as $x_w$, a side feature between the node $v$ and the node $w$ can be represented as $e_{vw}$, node information of the node $v$ can be represented as $m_v$, and a latent vector of the node $v$ can be represented as $h_v$. A process of calculating the node information of each node in the connection graph structure through the graph encoder (including a plurality of cascaded hidden layers) may include:

According to a node feature (denoted as $x_v$) of a first node (the first node is any node in the connection graph structure, such as a node $v$) in the connection graph structure, a node feature (denoted as $x_w$) of a second node (the second node is a neighbor node of the first node in the connection graph structure, such as a node $w$) in the connection graph structure, and side information (denoted as $h_{kv}^t$) between another node (such as a node $k$) in the neighbor nodes of the first node (excluding the second node) and the first node in a first hidden layer (assumed to be a hidden layer $t$), information (denoted as $m_{vw}^{t+1}$) between the first node and the second node in a second hidden layer (assumed to be a hidden layer $t+1$) is determined.

Then, side information (denoted as $h_{vw}^{t+1}$) between the first node and the second node in the second hidden layer is determined according to side information $h_{kv}^t$ between the first node and another node in the first hidden layer and information $m_{vw}^{t+1}$ between the first node and the second node in the second hidden layer. Side information in an initial hidden layer between two nodes in the connection graph structure (for example, side information of the node $v$ and the node $w$ in the initial hidden layer can be represented as $h_{vw}^0$) is obtained based on a node feature of one of the two nodes, and a side feature between the two nodes. On the basis of the above calculation, side information corresponding to each node in all hidden layers is summed to obtain the node information of each node.

In the embodiments of this application, the foregoing $m_{vw}^{t+1}$ may be obtained through the following formula (1):

$$m_{vw}^{t+1} = \sum_{k \in \{N(v)\backslash w\}} f_t\left(x_v, x_w, h_{kv}^t\right)$$

Formula (1)

In the above formula (1), $k \in \{N(v)\backslash w\}$ indicates that a node $k$ is a node other than a node $w$ in neighbor nodes $N(v)$ of a node $v$. $f_t(\cdot)$ represents an aggregation process. The aggregation process may be concatenating variables (i.e. $x_v$, $x_w$, and $h_{kv}^t$) (similar to a cat($\cdot$) function described below), or summing or averaging after the variables (i.e. $x_v$, $x_w$, and $h_{kv}^t$) are mapped to the same dimension, or combining the variables (i.e. $x_v$, $x_w$, and $h_{kv}^t$) in other forms.

In the embodiments of this application, the foregoing $h_{vw}^{t+1}$ may be obtained through the following formula (2):

$$h_{vw}^{t+1} = g_t(h_{vw}^t, m_{vw}^{t+1}) \quad \text{Formula (2)}$$

In the above formula (2), $g_t(\cdot)$ represents an update process. The update process may be simple accumulation or averaging, or may be a calculation form of a gated recurrent unit (GRU). If it is the calculation form of the GRU, then $h_{vw}^t$ is hidden layer input of the GRU, and $m_{vw}^{t+1}$ is actual input of the GRU.

In the embodiments of this application, the foregoing $h_{vw}^0$ may be obtained through the following formula (3):

$$h_{vw}^0 = \tau\left(W \cdot \text{cat}\left(x_v, e_{vw}\right)\right) \quad \text{Formula (3)}$$

In the above formula (3), $\tau(\cdot)$ represents a rectified linear unit (ReLU). $W$ represents a parameter to be learned. cat($\cdot$) indicates that two vectors are concatenated to form a longer vector. For example, a 3-dimensional vector is concatenated with a 5-dimensional vector to form an 8-dimensional vector.

In the embodiments of this application, assuming that node information of a node $v$ is $m_v$, the node information $m_v$ can be obtained through the following formula (4):

$$m_v = \sum_{k \in N(v)} h_{kv}^T \quad \text{Formula (4)}$$

In the above formula (4), $h_{kv}^T$ represents side information of a node $k$ and a node $v$ in all hidden layers. $k \in N(v)$ indicates that the node $k$ is a node in neighbor nodes $N(v)$ of the node $v$. Because there is side information only between neighbor nodes, $m_v$ is actually summing up side information of nodes $v$ in all hidden layers.

Step S320a. Generate latent vectors of each node according to the node information of each node and node features of each node.

In the embodiments of this application, using the foregoing example for description, after the node information $m_v$ of the node $v$ is obtained, a latent vector $h_v$ of the node $v$ can be generated according to the node information $m_v$ of the node $v$ and the node feature $x_v$ of the node $v$. For example, it can be obtained through the following formula (5):

$$h_v = \tau\left(W_a \cdot \text{cat}\left(x_v, m_v\right)\right) \quad \text{Formula (5)}$$

In the above formula (5), $\tau(\cdot)$ represents the ReLU function. $W_a$ represents a parameter to be learned. cat($\cdot$) indicates that two vectors are concatenated to form a longer vector.

Step S330a. Generate the atomic latent vector corresponding to the reference drug molecule according to the latent vectors of each node and the atom included in the reference drug molecule.

In an embodiment, after the latent vectors of each node are obtained, the atomic latent vector corresponding to the reference drug molecule can be represented through a matrix. That is, the latent vectors of each node are arranged in a matrix (such as an H matrix) in a row-column manner to represent the atomic latent vector corresponding to the reference drug molecule.

The foregoing formula (1) to formula (5) are only exemplary. In another embodiment of this application, the foregoing formula (1) to formula (5) may further be deformed appropriately (e.g., by increasing multiple, decreasing multiple, increasing certain value, decreasing certain value, etc.) to obtain a new calculation formula.

As shown in FIG. 2, in Step S220, atom masking processing is performed on the atomic latent vector corresponding to the reference drug molecule to obtain the scaffold latent vector and the sidechain latent vector included in the atomic latent vector.

In the embodiments of this application, after the atomic latent vector corresponding to the reference drug molecule is obtained, the process of performing atom masking processing on the atomic latent vector corresponding to the reference drug molecule may be shown in FIG. 3B, and include step S310b, step S320b, and step S330b. The detail is described as follows:

Step S310b. Determine a bit vector corresponding to the reference drug molecule. The length of the bit vector is the same as the number of atoms included in the reference drug molecule, and the bit value corresponding to a scaffold atom in the bit vector is a first value.

For example, in the foregoing embodiments, the first value may be 1. That is, if an atom belongs to the scaffold atom, a corresponding bit value in the bit vector is 1. If an atom belongs to a sidechain atom, a corresponding bit value in the bit vector is 0. In this case, the bit vector may be represented as a matrix $S_{sca}$ shown in the following formula (6):

$$S_{sca} = [1, i \in scaffold \ ; \ 0, i \notin scaffold] \quad \text{Formula (6)}$$

In the foregoing formula (6), $i \in scaffold$ indicates that an atom $i$ belongs to the scaffold atom. $i \notin scaffold$ indicates that the atom $i$ does not belong to the scaffold atom.

In the embodiments of this application, the foregoing bit vector may be preset, and is used for indicating which atoms in the reference drug molecule belong to the scaffold atoms and which atoms belong to the sidechain atoms. For example, the scaffold atom and the sidechain atom in the reference drug molecule may be determined by searching and matching in the reference drug molecule in a structural search manner based on a scaffold that needs to be transitioned (replaced) in the reference drug molecule, to obtain the foregoing bit vector.

In the embodiments of this application, the foregoing bit vector may be preset according to a set of scaffold determination rules. The scaffold determination rules may include a plurality of requirements, such as a requirement for a number of heavy atoms of a scaffold, a requirement for a number of scaffold rings, and the like. This is not limited in the embodiments of this application. For a drug molecule, a scaffold part in the drug molecule can be automatically detected according to the scaffold determination rule, and then the bit vector can be generated according to the scaffold part and a part other than the scaffold part (i.e., a sidechain part) in the drug molecule.

Step S320b. Filter an atomic latent vector corresponding to the reference drug molecule according to the bit vector to obtain a latent vector of the scaffold atom and a latent vector of the sidechain atom.

In the embodiments of this application, it is assumed that the atomic latent vector corresponding to the reference drug molecule (i.e., a latent vector of an original atom) is represented as $H_{node}$, the latent vector of scaffold atom selected from the atomic latent vector corresponding to the reference drug molecule can be represented as $H_{node}[S_{sca}]$, and the latent vector of the sidechain atom selected from the atomic latent vector corresponding to the reference drug molecule can be represented as $H_{node}[\overline{S}_{sca}]$.

Step S330b. Perform multi-head attention processing on the latent vector of the scaffold atom to obtain the scaffold latent vector, and perform multi-head attention processing on the latent vector of the sidechain atom to obtain the sidechain latent vector.

In the embodiments of this application, a multi-head attention mechanism is to determine a score (i.e., weight) corresponding to the latent vector of each atom (scaffold atom and sidechain atom), and then calculate the scaffold latent vector and the sidechain latent vector accordingly.

For example, assuming that the atomic latent vector corresponding to the reference drug molecule (i.e., the latent vector of the original atom) is represented as $H_{node}$. The scaffold latent vector $Z_{sca}$ in the embodiments of this application can be expressed by the following formula (7), and the sidechain latent vector $Z_{sc}$ can be expressed by the following formula (8):

$$Z_{sca} = \text{softmax}\left(W_1 \cdot \tanh\left(W_2, H_{node}^{\mathrm{T}}[S_{sca}]\right)\right) \cdot H_{node}[S_{sca}] \quad \text{Formula (7)}$$

$$Z_{sc} = \text{softmax}\left(W_1 \cdot \tanh\left(W_2, H_{node}^{\mathrm{T}}[\overline{S}_{sca}]\right)\right) \cdot H_{node}[\overline{S}_{sca}] \quad \text{Formula (8)}$$

In the foregoing formula (7) and formula (8), softmax(·) function realizes a function of the multi-head attention

mechanism. $W_1$ and $W_2$ are all learnable parameters. $H^{\mathrm{T}}_{node}$ represents transposition of $H_{node}$.

Certainly, the foregoing formula (7) and formula (8) are only exemplary. In another embodiment of this application, the foregoing formula (7) and formula (8) may further be deformed appropriately (e.g., by increasing multiple, decreasing multiple, increasing certain value, decreasing certain value, etc.) to obtain a new calculation formula.

Referring to FIG. 2, in Step S230, the target scaffold latent vector having the target transition degree (also referred to as a specified transition degree) between the target scaffold latent vector and the scaffold latent vector is generated according to the spatial distribution of the scaffold latent vector.

In the embodiments of this application, the spatial distribution of the scaffold latent vector may be a Gaussian mixture distribution, a von Misses-Fisher Mixture (vMFM) distribution, and the like. The following uses the Gaussian mixture distribution as an example for description:

In the embodiments of this application, a plurality of scaffold clusters may be preset, and cluster centers of each scaffold cluster in the plurality of scaffold clusters fit the Gaussian mixture distribution. For example, the plurality of scaffold clusters may be obtained by clustering a scaffold of the existing molecule through a scaffold clustering algorithm (i.e., clustering algorithm), and a cluster center of a scaffold cluster is fitted to the Gaussian mixture distribution, so that scaffold latent vectors included in a scaffold cluster all belong to a spatial distribution corresponding to the Gaussian mixture distribution.

In this case, after the scaffold latent vector is obtained, a first distance between the scaffold latent vector and the cluster center of each scaffold cluster can be determined, then a target scaffold cluster to which a scaffold of the reference drug molecule belongs can be determined according to the first distance, and then a Gaussian mixture distribution to which the scaffold latent vector belongs can be determined according to a cluster center of the target scaffold cluster.

For example, a cluster center of $m^{th}$ scaffold cluster can be represented as $(\mu_m, \sigma_m)$. $\mu_m$ represents a center of a cluster and $\sigma_m$ represents a standard deviation. For general expression, assuming that a scaffold latent vector corresponding to $i^{th}$ drug molecule is represented as $Z_{sca,i}$, a distance $d_i$ between the scaffold latent vector $Z_{sca,i}$ corresponding to the $i^{th}$ drug molecule and the cluster center of the $m^{th}$ scaffold cluster can be expressed as formula (9):

$$d_i = \frac{1}{2}\left(Z_{sca,i} - \mu_m\right)\sum_m^{-1}\left(Z_{sca,i} - \mu_m\right)^{\mathrm{T}} \qquad \text{Formula (9)}$$

Based on the distance $d_i$ between the scaffold latent vector $Z_{sca,i}$ corresponding to the $i^{th}$ drug molecule and the cluster center of the $m^{th}$ scaffold cluster calculated through the foregoing formula (9), a nearest target scaffold cluster (denoted as $c_i$) between the cluster center and the scaffold latent vector $Z_{sca,i}$ corresponding to the $i^{th}$ drug molecule can be selected, and then a Gaussian mixture distribution to which a scaffold cluster vector belongs can be determined based on the cluster center $(\mu_i, \sigma_i)$ of the target scaffold cluster $c_i$.

After the spatial distribution of the scaffold latent vector corresponding to the reference drug molecule is obtained and the target scaffold cluster is determined, the target scaffold latent vector having the specified transition degree between the target scaffold latent vector and the scaffold latent vector corresponding to the reference drug molecule can be generated according to a requirement, which is described in detail as follows:

As shown in FIG. 3C, a process of generating the target scaffold latent vector having the target transition degree according to the embodiments of this application may include step S310c and step S320c, which are described in detail as follows:

Step S310c. Perform random sampling processing on the target scaffold cluster according to the target transition degree to obtain an offset corresponding to the target transition degree.

Step S320c. Add the scaffold latent vector of the reference drug molecule and the offset corresponding to the target transition degree to obtain the target scaffold latent vector.

For example, the specified transition degree may be scaffold crawling, scaffold hopping, or scaffold leaping. The following describes the three transition methods.

In this embodiment of this application, when the specified transition degree is a first transition degree, a first offset can be obtained according to a product of a variance of the target scaffold cluster and a first vector obtained by random sampling, and then the first offset and the scaffold latent vector corresponding to the reference drug molecule are added to generate the target scaffold latent vector. For example, the first transition degree may be the scaffold crawling.

Specifically, assuming that the target scaffold cluster is represented as $c_i$, and the scaffold latent vector corresponding to the reference drug molecule is represented as $Z_{sca}$, the generated target scaffold latent vector having the first transition degree can be represented through the following formula (10):

$$Z_{new\_sca} = Z_{sca} + \sigma^2 (c_i) \times N(0,1) \qquad \text{Formula (10)}$$

In the foregoing formula (10), $Z_{new\_sca}$ represents a generated target scaffold latent vector, $\sigma^2(c_i)$ represents a variance of a Gaussian mixture distribution that a cluster center of a target scaffold cluster $c_i$ fits, and N(0,1) represents random sampling based on a distribution with a mean of 0 and a standard deviation of 1.

In this embodiment of this application, when the specified transition degree is a second transition degree, a first scaffold cluster whose distance from the cluster center of the target scaffold cluster is less than or equal to a first set value can be selected from a plurality of scaffold clusters. Then, a second offset is generated according to a product of a variance of the first scaffold cluster and a second vector obtained by random sampling, the cluster center of the target scaffold cluster, and the cluster center of the first scaffold cluster, and then the second offset and the scaffold latent vector corresponding to the reference drug molecule are added to generate the target scaffold latent vector. For example, the second transition degree may be the scaffold hopping.

For example, assuming that the target scaffold cluster is represented as $c_i$, the first scaffold cluster is represented as $c_j$, and the scaffold latent vector corresponding to the reference drug molecule is represented as $Z_{sca}$, the generated target scaffold latent vector having the second transition degree can be represented through the following formula (11):

$$Z_{new\_sca} = Z_{sca} - \mu(c_i) + \mu(c_j) + \sigma^2(c_j) \times N(0,1)$$

$$c_j = \pi \left( \left\{ c_k \, \middle\| \, \mu(c_i) - \mu(c_k) \right\| \leq \delta', j \neq i \right\} \right) \qquad \text{Formula (11)}$$

In the above formula (11), $Z_{new\_sca}$ represents a generated target scaffold latent vector, $\sigma^2(c_j)$ represents a variance of a Gaussian mixture distribution that a cluster center of a first scaffold cluster $c_j$ fits, N(0,1) represents random sampling based on a distribution with a mean of 0 and a standard deviation of 1, $\mu(c_i)$ represents a center of the target scaffold cluster $c_i$, $\mu(c_j)$ represents a center of the first scaffold cluster $c_j$, $\mu(c_k)$ represents a center of the scaffold cluster $c_k$, $\delta$ represents the first set value, $\pi(\cdot)$ represents a multi-nominal matrix sample, and $c_j = \pi(\{c_k \| \mu(c_i)-\mu(c_k) \| \leq \delta, j \neq i\})$ indicates that a scaffold cluster $c_j$ whose distance from the cluster center of the scaffold cluster $c_i$ is less than or equal to $\delta$ is found.

In this embodiment of this application, if the specified transition degree is a third transition degree, a second scaffold cluster whose distance from the cluster center of the target scaffold cluster is greater than or equal to a second set value can be selected from a plurality of scaffold clusters. Then, a third offset is generated according to a product of a variance of the second scaffold cluster and a third vector obtained by random sampling, the cluster center of the target scaffold cluster, and the cluster center of the second scaffold cluster, and then the third offset and the scaffold latent vector corresponding to the reference drug molecule are added to generate the target scaffold latent vector. For example, the third transition degree may be the scaffold leaping.

For example, assuming that the target scaffold cluster is represented as $c_i$, the second scaffold cluster is represented as $c_j$, and the scaffold latent vector corresponding to the reference drug molecule is represented as $Z_{sca}$, the generated target scaffold latent vector having the third transition degree can be represented through the following formula (12):

$$Z_{new\_sca} = Z_{sca} - \mu(c_i) + \mu(c_j) + \sigma^2(c_j) \times N(0,1)$$

$$c_j = \pi \left( \left\{ c_k \, \middle\| \, \mu(c_i) - \mu(c_k) \right\| \geq \Delta, j \neq i \right\} \right) \qquad \text{Formula (12)}$$

In the foregoing formula (12), $Z_{new\_sca}$ represents a generated target scaffold latent vector, $\sigma^2(c_j)$ represents a variance of a Gaussian mixture distribution that a cluster center of a second scaffold cluster $c_j$ fits, N(0,1) represents random sampling based on a distribution with a mean of 0 and a standard deviation of 1, $\mu(c_i)$ represents a center of the target scaffold cluster $c_i$, $\mu(c_j)$ represents a center of the second scaffold cluster $c_j$, $\mu(c_k)$ represents a center of the scaffold cluster $c_k$, $\Delta$ represents the second set value, $\pi(\cdot)$ represents a multi-nominal matrix sample, and $c_j = \pi(\{c_k\|\|\mu(c_i)-\mu(c_k)\| \geq \Delta, j \neq i\})$ indicates that a scaffold cluster $c_j$ whose distance from the cluster center of the scaffold cluster $c_i$ is greater than or equal to $\Delta$ is found.

The foregoing formula (9) to formula (12) are only exemplary. In another embodiment of this application, the foregoing formula (9) to formula (12) may further be deformed appropriately (e.g., by increasing multiple, decreasing multiple, increasing certain value, decreasing certain value, etc.) to obtain a new calculation formula.

Referring to FIG. 2, in step S240, a transitioned drug molecule is generated according to the target scaffold latent vector and the sidechain latent vector.

In this embodiment of this application, the scaffold latent vector in the reference drug molecule can be replaced by the target scaffold latent vector and combined with the sidechain latent vector to obtain the transitioned drug molecule.

In this embodiment of this application, a target and a target activity value of a specified reference drug molecule can further be obtained, and then the transitioned drug molecule is generated according to the target scaffold latent vector, the sidechain latent vector, the target and the target activity value of the specified reference drug molecule. In the technical solutions of this embodiment, activity of the generated drug molecule can be limited through the target and the target activity value of the reference drug molecule.

In this embodiment of this application, after the transitioned drug molecule is generated, the generated drug molecule may further be filtered. For example, molecular filtration processing of physical and chemical properties can be performed on the transitioned drug molecule to obtain a drug-like drug molecule. Then a eutectic structure corresponding to the reference drug molecule is obtained, and the drug-like drug molecule is docked to the eutectic structure, so as to remove a drug molecule mismatched with the eutectic structure through a binding mode of the drug-like drug molecule and the eutectic structure, to obtain the filtered drug molecule. In addition, a compound can be synthesized and verified according to docking of the filtered drug molecule and the eutectic structure.

For example, the eutectic structure corresponding to the reference drug molecule may be a eutectic structure of the reference drug molecule or a eutectic structure of compounds of the reference drug molecule in the same series. The drug molecule mismatched with the eutectic structure may be a drug molecule whose configuration is obviously unreasonable after docking.

In this embodiment of this application, relevant processing in the foregoing embodiments can be performed through a machine learning model. In a process of training the machine learning model, according to the technical solutions of the embodiments of this application, a solution of generating a loss function through a cross-entropy loss and a predicted loss of the machine learning model for a sample molecule. The following respectively describes how to obtain the cross-entropy loss and the predicted loss:

In this embodiment of this application, when the cross-entropy loss is calculated, a sample scaffold latent vector corresponding to the sample molecule can be obtained, and a plurality of scaffold clusters (the plurality of scaffold clusters can be the same as the plurality of scaffold clusters used in processing the reference drug molecule) can be obtained. Cluster centers of each scaffold cluster in the plurality of scaffold clusters fit the Gaussian mixture distribution. Then a second distance between the sample scaffold latent vector of the sample molecule and the cluster centers of each scaffold cluster is determined, a scaffold cluster to which a sample scaffold of the sample molecule belongs is determined according to the second distance, and a distance-based cross-entropy loss is generated according to the distance between the sample scaffold latent vector and the cluster center of the scaffold cluster to which the sample scaffold belongs.

A solution of obtaining the sample scaffold latent vector corresponding to the sample molecule is the same as a solution of obtaining the scaffold latent vector corresponding to the reference drug molecule, and details are not repeated herein. In addition, a formula for calculating the second distance between the sample scaffold latent vector of the sample molecule and the cluster center of each scaffold cluster may also be calculated through the foregoing formula (9).

For example, the foregoing formula (9) is used as an example for description (because related calculation formulas and processing methods of the sample molecule and the reference drug molecule are the same, the formula (9) can be used for calculating the distance between the scaffold latent vector corresponding to the reference drug molecule and the cluster center of the scaffold cluster, and also can be used for calculating the distance between the scaffold latent vector corresponding to the sample molecule and the cluster center of the scaffold cluster), assuming that a distance between a sample scaffold latent vector corresponding to $i^{th}$ drug molecule (which can be understood as $i^{th}$ sample molecule herein) and the cluster center of the scaffold cluster to which the sample scaffold belongs is represented as $d_i$, a certain deflection can be added on the basis of $d_i$ to improve accuracy of model training, as shown in formula (13):

$$d_{adj,i} = d_i + \text{onehot}\left(c_i\right) \times \delta d_i \qquad \text{Formula (13)}$$

In formula (13), $d_{adj,i}$ represents a distance after the deflection is added on the basis of $d_i$, onehot($\cdot$) represents a function of one-hot encoding, $c_i$ is used for representing a scaffold cluster to which a sample scaffold of $i^{th}$ sample molecule belongs, and $\delta$ represents a parameter.

In this embodiment of this application, after $d_{adj,i}$ is obtained, a distance-based cross-entropy loss $L_{cls}$ can be generated according to the following formula (14):

$$L_{cls} = \text{cross\_entropy}\left(-d_{adj,i} + \frac{1}{2}\log\left(\sum_m \sigma_m\right), c_i\right) \qquad \text{Formula (14)}$$

In formula (14), $\sigma_m$ represents a standard deviation of a Gaussian mixture distribution that a cluster center of $m^{th}$ scaffold cluster fits.

In the embodiments of this application, the machine learning model includes a decoder, after the sample scaffold latent vector and the sample sidechain latent vector corresponding to the sample molecule are obtained through the machine learning model, the sample scaffold latent vector, the sample sidechain latent vector, and the target molecule corresponding to the sample molecule are inputted into the decoder, and then a predicted loss of the machine learning model is calculated according to output of the decoder and the target molecule.

The target molecule is a molecule expected to be generated after the sample molecule is processed. A solution of obtaining the sample scaffold latent vector and the sample sidechain latent vector corresponding to the sample molecule is similar to a solution of obtaining the scaffold latent vector and the sidechain latent vector corresponding to the reference drug molecule, and details are not repeated herein.

In the embodiments of this application, after the cross-entropy loss and the predicted loss of the machine learning model are calculated, a loss function of the machine learning model can be generated according to the cross-entropy loss and the predicted loss of the machine learning model, and then a parameter of the machine learning model is adjusted based on the loss function. For example, a loss function $L$ of the machine learning model may be generated through the following formula (15):

$$L = L_{recon} + \beta L_{cls} \qquad \text{Formula (15)}$$

In the above formula (15), $L_{recon}$ represents a predicted loss of the machine learning model, and $\beta$ represents a hyperparameter for adjusting a weight between two losses.

A purpose of training the machine learning model is to minimize the foregoing loss function $L$. A purpose of setting the cross-entropy loss $L_{cls}$ is to ensure that each scaffold latent vector determined by the machine learning model is near a center of the scaffold cluster to which it belongs to a largest extent after the machine learning model is trained. A purpose of setting the predicted loss $L_{recon}$ is to ensure that the machine learning model can find a better target scaffold latent vector to a largest extent after the machine learning model is trained, and then ensure that a qualified drug molecule can be obtained.

After the machine learning model is trained, the reference drug molecule can be processed based on the machine learning model to obtain the transitioned drug molecule. In order to facilitate understanding of the technical solutions of the embodiments of this application, the following describes implementation details of the technical solutions of the embodiments of this application in detail with reference to FIG. 3 to FIG. 10:

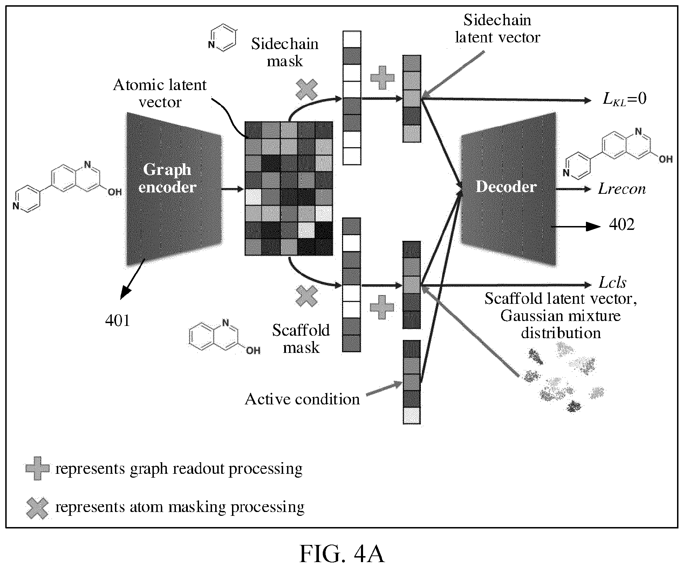

As shown in FIG. 4A, when the molecular scaffold transition is processed through the machine learning model, a model structure may include the following parts: a graph encoder 401, atom masking and graph readout, Gaussian mixture distribution (GM) fitting processing, and a decoder 402. The graph encoder is mainly configured to generate the atomic latent vector corresponding to the drug molecule. The atom masking and graph readout part is mainly used for obtaining the scaffold latent vector and the sidechain latent vector by atom masking processing. The Gaussian mixture distribution fitting processing is used for achieving the Gaussian mixture distribution of the scaffold latent vector, so as to implement processing of different transition degrees. The decoder is configured to output the drug molecule obtained after transition processing. The following respectively describes these parts in detail:

In the embodiments of this application, the graph encoder includes a directed message passing neural network (D-MPNN), which is a graph convolutional neural network. The graph convolutional neural network directly acts on a graph structure including a chemical structure. A fingerprint representation assigns a single fixed-length feature vector to a molecule. Unlike the fingerprint representation, a graph structure representation assigns a feature vector to each bond and atom in the chemical structure.

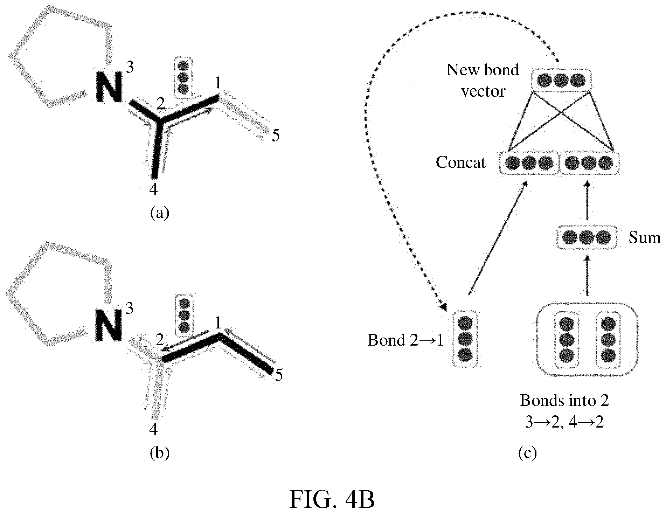

In short, the D-MPNN can be understood as a multi-step neural network, and each step is essentially a feedforward neural network. The neural network generates a set of latent representations for next input. A core of the D-MPNN is a message transmission step, in which a local substructure of a molecular graph is used for updating a latent vector. After the message transmission step, latent vectors from all edges are aggregated together into a single fixed-length latent vector, which is fed into the feedforward neural network to generate a prediction. As shown in FIG. 4B, each bond is represented through a pair of directed edges, and a message from an orange bond (i.e., 3→2 and 4→2 in (a)) in FIG. 4B(a) is used for notifying hidden state update of a red bond (i.e., 2→1 in (a)). A message from a green bond in (b) (i.e., 5→1 in (b)) is used for notifying hidden state update of a purple bond (i.e., 1→2 in (b)). An updating function of a hidden representation of a red bond (i.e., 2→1 in (a)) in (a) is represented through (c) in FIG. 4B, which is an iterative process that can be repeated for a plurality of times (e.g., 5 times). Concat shown in FIG. 4B is a strategy in deep learning, and can effectively process an input sample with a changeable size.

Before a drug molecule is input to the graph encoder, the drug molecule can be converted into a connection graph structure with a corresponding chemical bond and atom property on its side and point, so that the connection graph structure corresponding to the drug molecule can be represented as $G = (A, X, E)$. $A$ represents a connection matrix, $X$ represents a node feature, and $E$ represents a side feature. In the connection graph structure, a node represents an atom in the drug molecule. The node feature is used for representing an atomic feature in the drug molecule, which may include: atomic mass, atomic charge number, atomic type, valence state, whether an atom is in a ring, whether it is an atom in an aromatic ring, and the like. The side feature is used for representing a feature between atoms in the drug molecule, which may include: whether a side is single bond or double bond, whether the side is in the ring, whether the side is in the aromatic ring, and the like. On this basis, the connection graph structure is inputted to the D-MPNN for processing, which can be represented through the foregoing formulas (1) to (5). Finally, a latent vector of each node in the connection graph structure, that is, a latent vector of each atom in the drug molecule, is obtained. Furthermore, the atomic latent vector corresponding to the drug molecule can be represented through a matrix. That is, the latent vectors of each node are arranged in a matrix (such as an H matrix) in a row-column manner to represent the atomic latent vector corresponding to the drug molecule.

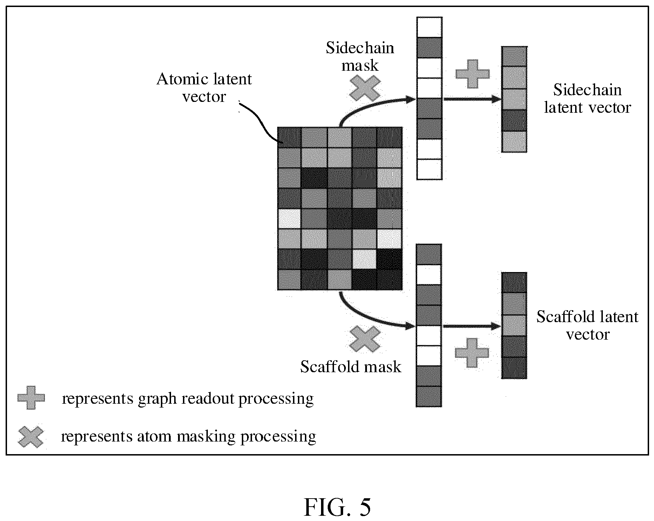

In the embodiments of this application, as shown in FIG. 5, the atom masking and graph readout part is mainly used for obtaining a latent vector representation of a scaffold and a sidechain, that is, the scaffold latent vector and the sidechain latent vector, by performing masking readout on the atom after latent vector representations of all atoms (i.e., the atomic latent vector corresponding to the drug molecule) are obtained.

For example, atom masking processing can be performed through a bit vector whose length is the same as a number of atoms included in the drug molecule. For example, the bit vector can be represented through the foregoing formula (6).

In the embodiments of this application, the graph readout is used for obtaining the scaffold latent vector and the sidechain latent vector, and a selective self-attention mechanism is used in the embodiments of this application. Assuming that an atomic latent vector of a drug molecule is $H_{node}$, a scaffold latent vector and a sidechain latent vector can be respectively calculated through the foregoing formula (7) and formula (8).

In the embodiments of this application, the Gaussian mixture distribution fitting processing mainly achieves the Gaussian mixture distribution of the scaffold latent vector, so as to implement processing of different transition degrees. For the sidechain, Gaussian distribution fitting may be performed or distribution hypothesis may not be performed. In the embodiments of this application, in order to better keep the sidechain unchanged, a hidden space of the sidechain can be processed through an autoencoder method without performing Gaussian distribution hypothesis.

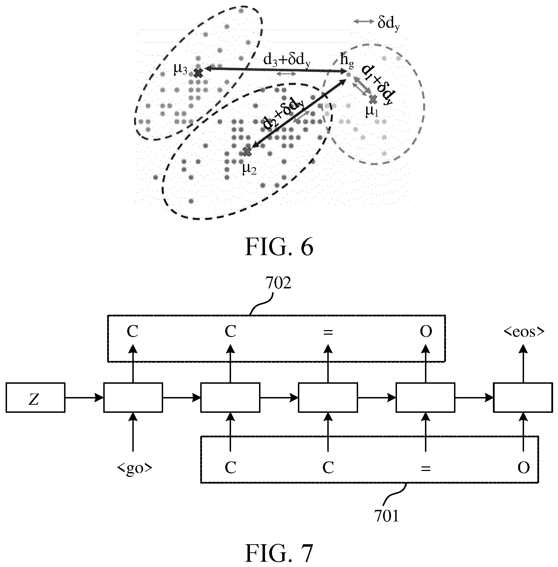

In the embodiments of this application, a scaffold of the existing molecule can be divided into $M$ different scaffold clusters through the scaffold clustering algorithm in advance. The existing molecule may be a sample molecule used for training the machine learning model, or a molecule selected from a molecular library, and these molecules are not limited to drug molecules. In a scaffold latent space, it is expected that points of the same scaffold cluster can be close to each other and points of different scaffold clusters can be far away from each other, so $M$ cluster centers of latent spaces can be set: $(\mu_m, \sigma_m)$. $\mu_m$ represents a center of a cluster, and $\sigma_m$ represents a standard deviation. Further, a distance $d_i$ between a scaffold latent vector $Z_{sca,i}$ corresponding to $i$th drug molecule and a cluster center of $m$th scaffold cluster can be calculated through the foregoing formula (9). In addition, a deflection can be added to the distance $d_i$ through the foregoing formula (13), and a representation method of the distance after the deflection is added can be shown in FIG. 6. After the distance is calculated, a distance-based cross-entropy loss $L_{cls}$ can be calculated through the foregoing formula (14).

In the embodiments of this application, the decoder can be a SMILES decoder, that is, a representation of a latent layer is decoded into a SMILES instead of a graph. The SMILES can be understood as a spanning tree of the graph expanded according to a rule, and each drug molecule can have a corresponding canonical SMILES, so it is proper for the decoder to use the SMILES. As shown in FIG. 7, the decoder can follow a teacher forcing mode. A working principle of the teacher forcing mode is using ground truth of a training data set as input x(t+1) of a next moment at a moment t of a training process, instead of using output of a previous moment of the model. In FIG. 7, 701 is an input part of the ground truth, and 702 is an output part of the model.

In the embodiments of this application, a loss reconstruction (i.e., the predicted loss) is performed on a final output result of an encoder with a correct answer (i.e., the ground truth) once to obtain $L_{recon}$. A loss function of the model includes a reconstruction loss and a cross-entropy loss, which can be referred to the foregoing formula (15).

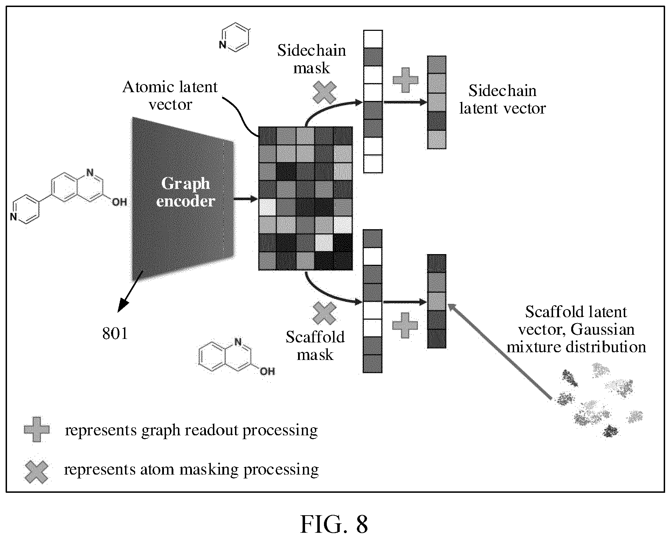

After the model is trained, the model can be used for molecular generation. In a process of the molecular generation, a molecule is needed to be inputted as a reference drug molecule, and the reference drug molecule is a drug molecule requiring scaffold replacement. In addition, a scaffold that needs to be replaced in the reference drug molecule can also be marked. After a structure of the reference drug molecule is converted into a connection graph structure and inputted into the model, the model can obtain a scaffold latent vector and a sidechain latent vector corresponding to the reference drug molecule. As shown in FIG. 8, functions of a graph encoder 801 and a graph encoder 401 in FIG. 4A are the same, and a processing process is similar to the related contents described in the foregoing embodiments. Details are not repeated herein.

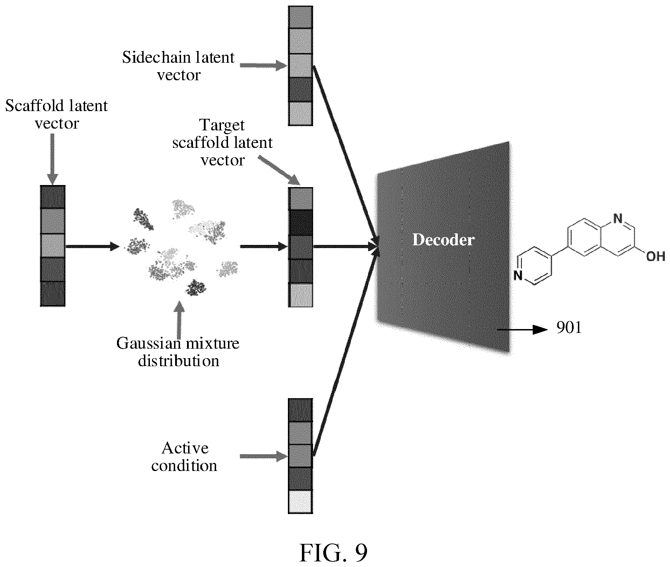

In the embodiments of this application, after the scaffold latent vector is obtained, a process of the molecular generation is slightly different from that of the model training. In the process of molecular generation, resampling processing is required when the target scaffold latent vector is obtained. A process of decoding processing is shown in FIG. 9, and functions of a decoder 901 and a decoder 402 in FIG. 4A are the same. The sidechain latent vector remains unchanged and is not sampled. Because of the model training, the scaffold latent vector shows a Gaussian mixture distribution state, and the distribution state is convenient for performing scaffold transition processing.

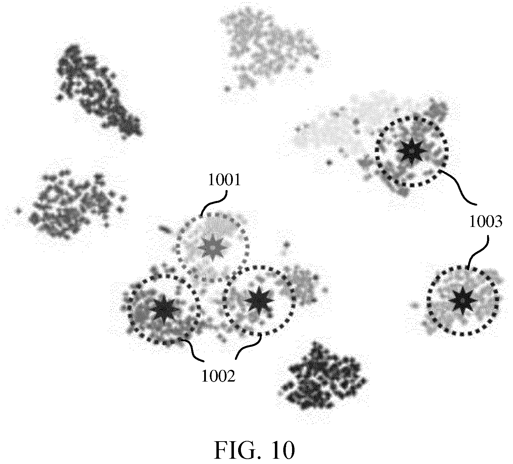

In the embodiments of this application, according to a condition of a transition degree, transition methods can be divided into the following three types: scaffold crawling, scaffold hopping, and scaffold leaping. As shown in FIG. 10, the scaffold crawling is a slightest transition, and has a minimal molecular change after transition. The scaffold latent vector is sampled from scaffold clusters having the same reference drug molecules (cluster 1001 in FIG. 10), and a target scaffold latent vector (i.e., a newly generated scaffold latent vector) corresponding to a new sampling point can be represented through the foregoing formula (10).

The scaffold hopping is a large transition, and has a large molecular scaffold change after transition. The scaffold latent vector is sampled from a nearby scaffold cluster of the reference drug molecule (cluster 1002 in FIG. 10), and a target scaffold latent vector (i.e., a newly generated scaffold latent vector) corresponding to a new sampling point can be represented through the foregoing formula (11).

The scaffold leaping is a transition to a largest extent, and has a largest molecular scaffold change after transition. The scaffold latent vector is sampled from a cluster (cluster 1003 in FIG. 10) far away from the scaffold cluster of the reference drug molecule, and a target scaffold latent vector (i.e., a newly generated scaffold latent vector) corresponding to a new sampling point can be represented through the foregoing formula (12).

Still referring to FIG. 9, while the sidechain latent vector and the target scaffold latent vector are obtained, it is necessary to input an active condition to the model, such as a target of the reference drug molecule and a pIC50 value corresponding to expectation. After the sidechain latent vector, the target scaffold latent vector, and the active condition are obtained, the model can generate a new transitioned drug molecule through the SMILES decoder.

In the embodiments of this application, the drug molecule generated after the scaffold transition can be filtered through the following two steps. A first step is molecular filtration based on physical and chemical properties, and its purpose is to ensure that a molecule in the following evaluation is drug-like. For example, it can be filtered through Lipinsiki five rules. A second step is to prepare a ligand for a drug-like molecule that meets a requirement of the physical and chemical properties, and enter a subsequent molecular docking step. Its purpose is to select a drug-like molecule with strong binding capability with the target.

For example, a crystal structure of the molecular docking can be searched from a protein data bank (PDB) database. For example, a eutectic structure of the reference drug molecule or its homologous compound can be selected, and it is ensured that a resolution is high and a protein structure near a binding pocket is complete. During docking, protein preparation is performed through molecular docking software, and then a molecule is docked back to a prepared crystal structure. Accuracy of configuration is determined through a binding mode. In addition, a molecular binding mode in a eutectic structure is also used as a template for molecular docking to analyze whether a binding mode of a molecule generated through AI is appropriate. According to the technical solutions of the embodiments, a molecule with an obviously inappropriate configuration can be removed through virtual filtration. Then, all the configurations retained in a previous step are docked with a molecule with higher precision, and then an obtained binding mode is re-scored through a 3D-convolutional neural network (CNN) method. A molecule whose 3D-CNN score is at least greater than 0.8 (the value is only an example) and binding mode of a key action site is not lost is selected for compound synthesis and verification.

In the foregoing embodiments, the graph encoder may further use Dual-MPNN. The SMILES decoder can be replaced by various natural language processing decoders, such as a grammar-variational autoencoder (VAE), a syntax directed-VAE (SD-VAE), and a decoding part of Transformer.

By using the technical solutions in the embodiments of this application, a scaffold latent vector can be mapped to a spatial distribution, so that a generated target scaffold latent vector can get rid of a design mindset of pharmaceutical experts, and good novelty can be achieved. In addition, the solution can be automatically executed through an electronic device, which reduces manpower and time costs.

The following describes apparatus embodiments of this application, which may be used for executing the method for processing a molecular scaffold transition in the foregoing embodiments of this application. For details not disclosed in the apparatus embodiments of this application, reference may be made to the foregoing embodiments of the method for processing a molecular scaffold transition of this application.

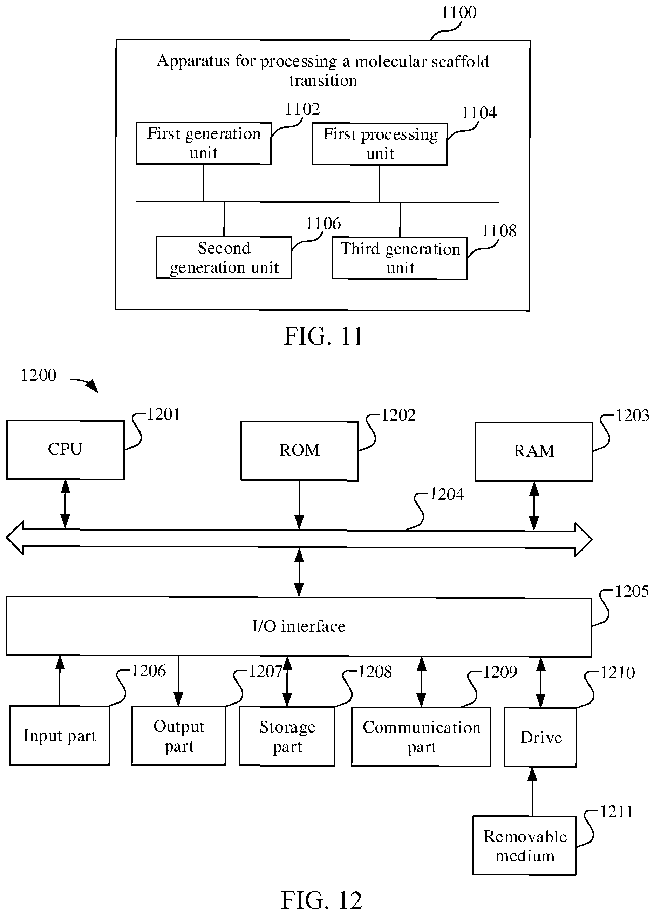

FIG. 11 is a block diagram of an apparatus for processing a molecular scaffold transition according to an embodiment of this application. The apparatus for processing a molecular scaffold transition may be arranged in a device having a calculation processing function, such as the server 130 shown in FIG. 1.

Referring to FIG. 11, an apparatus 1100 for processing a molecular scaffold transition according to the embodiments of this application includes: a first generation unit 1102, a first processing unit 1104, a second generation unit 1106, and a third generation unit 1108.

The first generation unit 1102 is configured to generate, according to a connection graph structure corresponding to a reference drug molecule, an atomic latent vector corresponding to the reference drug molecule. The first processing unit 1104 is configured to perform atom masking processing on the atomic latent vector to obtain a scaffold latent vector and a sidechain latent vector included in the atomic latent vector. The second generation unit 1106 is configured to generate a target scaffold latent vector with a target transition degree between the scaffold latent vector and the target scaffold latent vector according to a spatial distribution of the scaffold latent vector. The third generation unit 1108 is configured to generate a transitioned drug molecule according to the target scaffold latent vector and the sidechain latent vector.

In some embodiments, based on the foregoing solutions, a node in the connection graph structure represents an atom in the reference drug molecule. The first generation unit 1102 is configured to: determine node information of each node in the connection graph structure through a graph encoder according to a node feature and a side feature included in the connection graph structure. The node feature represents an atomic feature in the reference drug molecule. The side feature represents a feature between atoms in the reference drug molecule. The first generation unit 1102 is configured to generate latent vectors of each node according to the node information of each node and node features of each node; and generate the atomic latent vector corresponding to the reference drug molecule according to the latent vectors of each node and the atom included in the reference drug molecule.

In some embodiments, based on the foregoing solutions, the first generation unit 1102 is configured to: include a plurality of cascaded hidden layers through the graph encoder; and according to a node feature of a first node in the connection graph structure, a node feature of a second node in the connection graph structure, and side information between another node except the second node in neighbor nodes of the first node and the first node in a first hidden layer, determine information between the first node and the second node in a second hidden layer, the first node being any node in the connection graph structure, the second node being any neighbor node of the first node in the connection graph structure, and the second hidden layer being a next hidden layer of the first hidden layer; determine side information between the first node and the second node in the second hidden layer according to side information between the first node and another node in the first hidden layer and information between the first node and the second node in the second hidden layer, side information between two nodes in the connection graph structure in an initial hidden layer being obtained according to a node feature of one of the two nodes and a side feature between the two nodes; and sum side information corresponding to each node in the plurality of hidden layers to obtain the node information of each node.

In some embodiments, based on the foregoing solutions, the first processing unit 1104 is configured to: determine a bit vector corresponding to the reference drug molecule, a length of the bit vector is the same as a number of atoms included in the reference drug molecule, and a bit value corresponding to a scaffold atom in the bit vector being a first value; filter an atomic latent vector corresponding to the reference drug molecule according to the bit vector to obtain a latent vector of the scaffold atom and a latent vector of the sidechain atom; and perform multi-head attention processing on the latent vector of the scaffold atom to obtain the scaffold latent vector, and perform multi-head attention processing on the latent vector of the sidechain atom to obtain the sidechain latent vector.

In some embodiments, based on the foregoing solutions, the first processing unit 1104 is further configured to: obtain a plurality of scaffold clusters, cluster centers of each scaffold cluster in the plurality of scaffold clusters fitting a Gaussian mixture distribution; determine a first distance between the scaffold latent vector and the cluster centers of each scaffold cluster, and determine a target scaffold cluster to which a scaffold of the reference drug molecule belongs according to the first distance; and determine a Gaussian mixture distribution to which the scaffold latent vector belongs according to the cluster center of the target scaffold cluster.

In some embodiments, based on the foregoing solutions, the second generation unit 1106 is configured to: perform random sampling processing on the target scaffold cluster according to the target transition degree to obtain an offset corresponding to the target transition degree; and add the scaffold latent vector and the offset corresponding to the target transition degree to obtain the target scaffold latent vector.

In some embodiments, based on the foregoing solutions, the second generation unit 1106 is configured to: multiply, when the target transition degree is a first transition degree, a variance of the target scaffold cluster and a first vector obtained by random sampling to obtain a first offset, and use the first offset as an offset corresponding to the first transition degree, the first transition degree representing scaffold crawling.

In some embodiments, based on the foregoing solutions, the second generation unit 1106 is configured to: select a first scaffold cluster from the plurality of scaffold clusters when the target transition degree is a second transition degree, a distance between the first scaffold cluster and the cluster center of the target scaffold cluster being less than or equal to a first set value, and the second transition degree representing scaffold hopping; and generate a second offset according to a product of the variance of the first scaffold cluster and a second vector obtained by random sampling, the cluster center of the target scaffold cluster, and the cluster center of the first scaffold cluster, and use the second offset as an offset corresponding to the second transition degree.

In some embodiments, based on the foregoing solutions, the second generation unit 1106 is configured to: select a second scaffold cluster from the plurality of scaffold clusters when the target transition degree is a third transition degree, a distance between the second scaffold cluster and the cluster center of the target scaffold cluster being greater than or equal to a second set value; and generate a third offset according to a product of a variance of the second scaffold cluster and a third vector obtained by random sampling, a cluster center of the target scaffold cluster, and the cluster center of the second scaffold cluster, and use the third offset as an offset corresponding to the third transition degree.

In some embodiments, based on the foregoing solutions, the third generation unit 1108 is configured to: obtain a target and a target activity value of the specified reference drug molecule; and generate the transitioned drug molecule according to the target scaffold latent vector, the sidechain latent vector, the target and the target activity value of the specified reference drug molecule.

In some embodiments, based on the foregoing solutions, the apparatus 1100 further includes a second processing unit. The second processing unit is configured to: after the transitioned drug molecule is generated, perform molecular filtration processing of physicochemical property according to the transitioned drug molecule to obtain a drug-like drug molecule; obtain a eutectic structure corresponding to the reference drug molecule, and docking the drug-like drug molecule to the eutectic structure; remove a drug molecule that does not match the eutectic structure through a binding mode of the drug-like drug molecule and the eutectic structure to obtain a filtered drug molecule; and synthesize and verify a compound according to docking of the filtered drug molecule and the eutectic structure.

In some embodiments, based on the foregoing solutions, the method for processing a molecular scaffold transition is implemented through a machine learning model. The apparatus 1100 further includes: a third processing unit, configured to obtain a sample scaffold latent vector corresponding to a sample molecule, and obtain a plurality of scaffold clusters, cluster centers of each scaffold cluster in the plurality of scaffold clusters fitting a Gaussian mixture distribution; determine a second distance between the sample scaffold latent vector of the sample molecule and the cluster centers of each scaffold cluster, and determine a scaffold cluster to which a sample scaffold of the sample molecule belongs according to the second distance; generate a distance-based cross-entropy loss according to the distance between the sample scaffold latent vector and the cluster center of the scaffold cluster to which the sample scaffold belongs; generate a loss function of the machine learning model according to the cross-entropy loss and a predicted loss of the machine learning model for the sample molecule; and adjust a parameter of the machine learning model based on the loss function.

In some embodiments, based on the foregoing solutions, the machine learning model includes a decoder. The third processing unit is further configured to: input, after the sample scaffold latent vector and a sample sidechain latent vector corresponding to the sample molecule are obtained through the machine learning model, the sample scaffold latent vector, the sample sidechain latent vector, and a target molecule corresponding to the sample molecule to the decoder; and determine the predicted loss according to output of the decoder and the target molecule.

FIG. 12 is a schematic structural diagram of a computer system adapted to implement an electronic device according to an embodiment of this application.

The computer system 1200 of the electronic device shown in FIG. 12 is merely an example, and does not constitute any limitation on functions and use ranges of the embodiments of this application.

As shown in FIG. 12, the computer system 1200 includes a central processing unit (CPU) 1201 that can execute various appropriate actions and processes. For example, the computer system 1200 executes the methods described in the foregoing embodiments, according to a program stored in a read-only memory (ROM) 1202 or a program loaded into a random access memory (RAM) 1203 from a storage part 1208. The RAM 1203 further stores various programs and data required for operating the system. The CPU 1201, the ROM 1202, and the RAM 1203 are connected to each other through a bus 1204. An input/output (I/O) interface 1205 is also connected to the bus 1204.

The following components are connected to the I/O interface 1205: an input part 1206 including a keyboard and a mouse, etc.; an output part 1207 including a cathode ray tube (CRT), a liquid crystal display (LCD), a speaker, or the like; a storage part 1208 including hard disk, or the like; and a communication part 1209 including a network interface card such as a local area network (LAN) card, a modem, or the like. The communication part 1209 performs communication processing by using a network such as the Internet. A driver 1210 is also connected to the I/O interface 1205 as required. A removable medium 1211, such as a magnetic disk, an optical disc, a magneto-optical disk, or a semiconductor memory, is installed on the driver 1210 as required, so that a computer program read from the removable medium is installed into the storage part 1208 as required.

Particularly, according to an embodiment of this application, the processes described in the following by referring to the flowcharts may be implemented as computer software programs. For example, an embodiment of this application includes a computer program product. The computer program product includes a computer program stored in a computer-readable medium. The computer program includes a computer program used for executing a method shown in the flowchart. In such an embodiment, the computer program may be downloaded and installed through the communication part 1209 from a network, and/or installed from the removable medium 1211. When the computer program is executed by the CPU 1201, the various functions defined in the system of this application are executed.

The computer-readable medium shown in the embodiments of this application may be a computer-readable signal medium or a computer-readable storage medium or any combination of two. The computer-readable storage medium may be, for example, but is not limited to, an electrical, magnetic, optical, electromagnetic, infrared, or semiconductor system, apparatus, or device, or any combination thereof. More specific examples of the computer-readable storage medium may include, but are not limited to: an electrical connection having one or more wires, a portable computer magnetic disk, a hard disk, a random access memory (RAM), a read-only memory (ROM), an erasable programmable read-only memory (EPROM), a flash memory, an optical fiber, a compact disk read-only memory (CD-ROM), an optical storage device, a magnetic storage device, or any appropriate combination thereof. In this application, the computer-readable storage medium may be any tangible medium containing or storing a program, and the program may be used by or used in combination with an instruction execution system, an apparatus, or a device. In this application, a computer-readable signal medium may include a data signal in a baseband or propagated as a part of a carrier wave, the data signal carrying a computer-readable program. A data signal propagated in such a way may assume a plurality of forms, including, but not limited to, an electromagnetic signal, an optical signal, or any appropriate combination thereof. The computer-readable signal medium may be further any computer-readable medium in addition to a computer-readable storage medium. The computer-readable medium may send, propagate, or transmit a program that is used by or used in combination with an instruction execution system, apparatus, or device. The computer program included in the computer-readable storage medium may be transmitted using any suitable medium, including but not limited to: a wireless medium, a wired medium, or any suitable combination thereof.

The flowcharts and block diagrams in the accompanying drawings illustrate possible system architectures, functions, and operations that may be implemented by a system, a method, and a computer program product according to various embodiments of this application. Each box in a flowchart or a block diagram may represent a module, a program segment, or a part of code. The module, the program segment, or the part of code includes one or more executable instructions used for implementing designated logic functions. In some implementations used as substitutes, functions annotated in boxes may alternatively occur in a sequence different from that annotated in an accompanying drawing. For example, actually two boxes shown in succession may be performed basically in parallel, and sometimes the two boxes may be performed in a reverse sequence. This is determined by a related function. Each box in a block diagram and/or a flowchart and a combination of boxes in the block diagram and/or the flowchart may be implemented by using a dedicated hardware-based system configured to perform a specified function or operation, or may be implemented by using a combination of dedicated hardware and a computer instruction.

A related unit described in the embodiments of this application may be implemented in a software manner, or may be implemented in a hardware manner, and the unit described may also be set in a processor. Names of the units do not constitute a limitation on the units in a specific case.

In another aspect, the embodiments of this application further provide a computer-readable storage medium. The computer-readable storage medium may be included in the electronic device described in the above embodiments, or may exist alone without being assembled into the electronic device. The computer-readable storage medium carries one or more programs, the one or more programs, when executed by the electronic device, causing the electronic device to implement the method described in the foregoing embodiments.

Although a plurality of modules or units of a device configured to perform actions are discussed in the foregoing detailed description, such division is not mandatory. Actually, according to the implementations of this application, the features and functions of two or more modules or units described above may be specifically implemented in one module or unit. Conversely, features and functions of one module or unit described above may be further divided into a plurality of modules or units for implementation.

Through the descriptions of the foregoing implementations, a person skilled in the art easily understands that the exemplary implementations described herein may be implemented through software, or may be implemented through software located in combination with necessary hardware. Therefore, the technical solutions of the embodiments of this application may be implemented in a form of a software product. The software product may be stored in a non-volatile storage medium (which may be a CD-ROM, a USB flash drive, a removable hard disk, or the like) or on the network, including several instructions for instructing a computing device (which may be a personal computer, a server, a touch terminal, a network device, or the like) to execute the methods according to the embodiments of this application.

After considering the specification and practicing the disclosed embodiments, a person skilled in the art may easily conceive of other implementations of this application. This application is intended to cover any variations, uses or adaptive changes of this application. Such variations, uses or adaptive changes follow the general principles of this application, and include well-known knowledge and conventional technical means in the art that are not disclosed in this application.

It is to be understood that this application is not limited to the precise structures described above and shown in the accompanying drawings, and various modifications and changes can be made without departing from the scope of this application. The scope of this application is limited by the appended claims only.

## Claims

1. A method for processing molecular scaffold transitions, executable by an electronic device, the method comprising:

   generating, according to a connection graph structure corresponding to a reference drug molecule, an atomic latent vector corresponding to the reference drug molecule;
   performing atom masking processing on the atomic latent vector to obtain a scaffold latent vector and a sidechain latent vector included in the atomic latent vector;
   generating a target scaffold latent vector with a target transition degree between the scaffold latent vector and the target scaffold latent vector according to a spatial distribution of the scaffold latent vector; and
   generating a transitioned drug molecule according to the target scaffold latent vector and the sidechain latent vector.

2. The method for processing molecular scaffold transitions according to claim 1, wherein:

   a node in the connection graph structure represents an atom in the reference drug molecule; and
   generating, according to the connection graph structure corresponding to the reference drug molecule, the atomic latent vector corresponding to the reference drug molecule comprises:

   determining node information of each node in the connection graph structure through a graph encoder according to a node feature and a side feature included in the connection graph structure, the node feature representing an atomic feature in the reference drug molecule, and the side feature representing a feature between atoms in the reference drug molecule;
   generating latent vectors of each node according to the node information of each node and node features of each node; and
   generating the atomic latent vector corresponding to the reference drug molecule according to the latent vectors of each node and the atom included in the reference drug molecule.

3. The method for processing molecular scaffold transitions according to claim 2, wherein

   the graph encoder comprises a plurality of cascaded hidden layers; and
   determining the node information of each node in the connection graph structure through the graph encoder according to the node feature and the side feature included in the connection graph structure comprises:

   determining information between the first node and the second node in a second hidden layer according to a node feature of a first node in the connection graph structure, a node feature of a second node in the connection graph structure, and side information between another node excluding the second node in neighbor nodes of the first node and the first node in a first hidden layer, wherein the first node is any node in the connection graph structure, the second node is any neighbor node of the first node in the connection graph structure, and the second hidden layer is a hidden layer next to the first hidden layer;
   determining side information between the first node and the second node in the second hidden layer according to the side information between the first node and the another node in the first hidden layer and the information between the first node and the second node in the second hidden layer, wherein side information between two nodes in the connection graph structure in an initial hidden layer is obtained according to a node feature of one of the two nodes and a side feature between the two nodes; and summing side information corresponding to each node in the plurality of hidden layers to obtain the node information of each node.

4. The method for processing molecular scaffold transitions according to claim 1, wherein performing the atom masking processing on the atomic latent vector to obtain the scaffold latent vector and the sidechain latent vector included in the atomic latent vector comprises:

determining a bit vector corresponding to the reference drug molecule, the bit vector having a length that is the same as a number of atoms in the reference drug molecule, and a bit value corresponding to a scaffold atom in the bit vector being a first value; and
filtering an atomic latent vector corresponding to the reference drug molecule according to the bit vector to obtain a latent vector of the scaffold atom and a latent vector of the sidechain atom; and
performing multi-head attention processing on the latent vector of the scaffold atom to obtain the scaffold latent vector, and performing multi-head attention processing on the latent vector of the sidechain atom to obtain the sidechain latent vector.

5. The method for processing molecular scaffold transitions according to claim 1, further comprising before generating the target scaffold latent vector with the target transition degree between the scaffold latent vector and the target scaffold latent vector according to the spatial distribution of the scaffold latent vector:

obtaining a plurality of scaffold clusters, wherein cluster centers of each scaffold cluster in the plurality of scaffold clusters fit a Gaussian mixture distribution;
determining a first distance between the scaffold latent vector and the cluster centers of each scaffold cluster, and determining a target scaffold cluster to which a scaffold of the reference drug molecule belongs according to the first distance; and
determining a Gaussian mixture distribution to which the scaffold latent vector belongs according to the cluster center of the target scaffold cluster.

6. The method for processing molecular scaffold transitions according to claim 5, wherein generating the target scaffold latent vector with the target transition degree between the scaffold latent vector and the target scaffold latent vector according to the spatial distribution of the scaffold latent vector comprises:

performing random sampling processing on the target scaffold cluster according to the target transition degree to obtain an offset corresponding to the target transition degree; and
adding the scaffold latent vector and the offset corresponding to the target transition degree to obtain the target scaffold latent vector.

7. The method for processing molecular scaffold transitions according to claim 6, wherein performing the random sampling processing on the target scaffold cluster according to the target transition degree to obtain the offset corresponding to the target transition degree comprises:
multiplying, when the target transition degree is a first transition degree, a variance of the target scaffold cluster and a first vector obtained by random sampling to obtain a first offset, and using the first offset as an offset corresponding to the first transition degree, the first transition degree representing scaffold crawling.

8. The method for processing molecular scaffold transitions according to claim 6, wherein performing the random sampling processing on the target scaffold cluster according to the target transition degree to obtain the offset corresponding to the target transition degree comprises:

selecting a first scaffold cluster from the plurality of scaffold clusters when the target transition degree is a second transition degree, wherein a distance between the first scaffold cluster and the cluster center of the target scaffold cluster is less than or equal to a first set value and the second transition degree represents scaffold hopping; and
generating a second offset according to a product of the variance of the first scaffold cluster and a second vector obtained by random sampling, the cluster center of the target scaffold cluster, and the cluster center of the first scaffold cluster, and using the second offset as an offset corresponding to the second transition degree.

9. The method for processing molecular scaffold transitions according to claim 6, wherein performing the random sampling processing on the target scaffold cluster according to the target transition degree to obtain the offset corresponding to the target transition degree comprises:

selecting a second scaffold cluster from the plurality of scaffold clusters when the target transition degree is a third transition degree, a distance between the second scaffold cluster and the cluster center of the target scaffold cluster being greater than or equal to a second set value; and
generating a third offset according to a product of a variance of the second scaffold cluster and a third vector obtained by random sampling, a cluster center of the target scaffold cluster, and the cluster center of the second scaffold cluster, and using the third offset as an offset corresponding to the third transition degree.

10. The method for processing molecular scaffold transitions according to claim 1, wherein generating the transitioned drug molecule according to the target scaffold latent vector and the sidechain latent vector comprises:

obtaining a target and a target activity value of the specified reference drug molecule; and
generating the transitioned drug molecule according to the target scaffold latent vector, the sidechain latent vector, the target and the target activity value of the specified reference drug molecule.

11. The method for processing molecular scaffold transitions according to claim 1, further comprising after generating the transitioned drug molecule according to the target scaffold latent vector and the sidechain latent vector:

performing molecular filtration processing of physicochemical property according to the transitioned drug molecule to obtain a drug-like drug molecule;
obtaining a eutectic structure corresponding to the reference drug molecule, and docking the drug-like drug molecule to the eutectic structure;
removing a drug molecule that does not match the eutectic structure through a binding mode of the drug-like drug molecule and the eutectic structure to obtain a filtered drug molecule; and
synthesizing and verifying a compound according to docking of the filtered drug molecule and the eutectic structure.

12. The method for processing molecular scaffold transitions according to any one of claims 1 to 11, wherein

the method for processing molecular scaffold transitions is implemented through a machine learning model; and
the method further comprises:

obtaining a sample scaffold latent vector corresponding to a sample molecule, and obtaining a plurality of scaffold clusters, cluster centers of each scaffold cluster in the plurality of scaffold clusters fitting a Gaussian mixture distribution;
determining a second distance between the sample scaffold latent vector of the sample molecule and the cluster centers of each scaffold cluster, and determining a scaffold cluster to which a sample scaffold of the sample molecule belongs according to the second distance; and
generating a distance-based cross-entropy loss according to the distance between the sample scaffold latent vector and the cluster center of the scaffold cluster to which the sample scaffold belongs;
generating a loss function of the machine learning model according to the cross-entropy loss and a predicted loss of the machine learning model for the sample molecule; and
adjusting a parameter of the machine learning model based on the loss function.

13. The method for processing molecular scaffold transitions according to claim 12, wherein

the machine learning model comprises a decoder; and
the method further comprises:

inputting, after the sample scaffold latent vector and a sample sidechain latent vector corresponding to the sample molecule are obtained through the machine learning model, the sample scaffold latent vector, the sample sidechain latent vector, and a target molecule corresponding to the sample molecule to the decoder; and

determining the predicted loss according to output of the decoder and the target molecule.

14. An apparatus for processing molecular scaffold transitions, comprising:

a first generation unit, configured to generate, according to a connection graph structure corresponding to a reference drug molecule, an atomic latent vector corresponding to the reference drug molecule;
a first processing unit, configured to perform atom masking processing on the atomic latent vector to obtain a scaffold latent vector and a sidechain latent vector included in the atomic latent vector;
a second generation unit, configured to generate a target scaffold latent vector with a target transition degree between the scaffold latent vector and the target scaffold latent vector according to a spatial distribution of the scaffold latent vector; and
a third generation unit, configured to generate a transitioned drug molecule according to the target scaffold latent vector and the sidechain latent vector.

15. A computer-readable medium, storing a computer program, the computer program, when executed by a processor, implementing the method for processing molecular scaffold transitions according to any one of claims 1 to 13.

16. An electronic device, comprising:
one or more processors; and
memory storing one or more programs, the one or more programs comprising instructions that, when executed by the one or more processors, cause the one or more processors to implement the method for processing molecular scaffold transitions according to any one of claims 1 to 13.

17. A computer program product, comprising a computer program or instruction, the computer program or instruction causing a computer to perform the method for processing a molecular scaffold transition according to any one of claims 1 to 13.

FIG. 1

| |
|---|
| Generate, according to a connection graph structure corresponding to a reference drug molecule, an atomic latent vector corresponding to the reference drug molecule |

S210

| |
|---|
| Perform atom masking processing on the atomic latent vector corresponding to the reference drug molecule to obtain the scaffold latent vector and the sidechain latent vector included in the atomic latent vector |

S220

| |
|---|
| Generate a target scaffold latent vector with a target transition degree between the scaffold latent vector and the target scaffold latent vector according to a spatial distribution of the scaffold latent vector |

S230

| |
|---|
| Generate a transitioned drug molecule according to the target scaffold latent vector and the sidechain latent vector |

S240

FIG. 2

Determine node information of each node in the connection graph structure through a graph encoder according to a node feature and a side feature included in the connection graph structure

S310a

Generate latent vectors of each node according to the node information of each node and node features of each node

S320a

Generate the atomic latent vector corresponding to the reference drug molecule according to the latent vectors of each node and the atom included in the reference drug molecule

S330a

FIG. 3A

Determine a bit vector corresponding to the reference drug molecule, a length of the bit vector being the same as a number of atoms included in the reference drug molecule, and a bit value corresponding to a scaffold atom in the bit vector being a first value

S310b

Filter an atomic latent vector corresponding to the reference drug molecule according to the bit vector to obtain a latent vector of the scaffold atom and a latent vector of the sidechain atom

S320b

Perform multi-head attention processing on the latent vector of the scaffold atom to obtain the scaffold latent vector, and perform multi-head attention processing on the latent vector of the sidechain atom to obtain the sidechain latent vector

S330b

FIG. 3B

Perform random sampling processing on the target scaffold cluster according to the target transition degree to obtain an offset corresponding to the target transition degree

S310c

Add the scaffold latent vector of the reference drug molecule and the offset corresponding to the target transition degree to obtain the target scaffold latent vector

S320c

FIG. 3C

FIG. 4A

(a)

(b)

(c)

FIG. 4B

FIG. 5

FIG. 6

FIG. 7

FIG. 8

FIG. 9

FIG. 10

1100

Apparatus for processing a molecular scaffold transition

1102
First generation unit

1104
First processing unit

1106
Second generation unit

1108
Third generation unit

FIG. 11

1200

1201
CPU

1202
ROM

1203
RAM

1204

1205
I/O interface

1206
Input part

1207
Output part

1208
Storage part

1209
Communication part

1210
Drive

1211
Removable medium

FIG. 12

## INTERNATIONAL SEARCH REPORT

| International application No. |
| --- |
| **PCT/CN2022/078336** |

| A. | CLASSIFICATION OF SUBJECT MATTER |
| --- | --- |

G16B 15/30(2019.01)i

According to International Patent Classification (IPC) or to both national classification and IPC

| B. | FIELDS SEARCHED |
| --- | --- |

Minimum documentation searched (classification system followed by classification symbols)

G16B

Documentation searched other than minimum documentation to the extent that such documents are included in the fields searched

Electronic data base consulted during the international search (name of data base and, where practicable, search terms used)

CNPAT, CNKI, WPI, EPODOC, IEEE: 分子, 侧链, 图, 化合物, 编码器, 迁越, 迁跃, 隐藏, 支链, 越迁, 支架, 遮挡, 隐藏, 原子, 原子, 隐向量, 骨架, 遮蔽, 生成, 掩膜, 跳跃, 隐向量, 骨架跃迁, 先导物, 药物, 跃迁, 掩蔽屏蔽, molecule+, drug, mask +, generat+, GMVAE, implicit vector, side chain, side, figure, hopping, scaffold hopping, encoder, compound, graph, scaffold, crawling, molecule, atom, leaping, implicit vector, hidden vector

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 112201301 A (SHENZHEN XTAIPI TECHNOLOGY CO., LTD.) 08 January 2021 (2021-01-08) description, paragraphs [0038]-[0040], and figures 6-8 | 1-17 |
| A | CN 108205613 A (SOUTH CHINA UNIVERSITY OF TECHNOLOGY) 26 June 2018 (2018-06-26) entire document | 1-17 |
| A | CN 111209468 A (INNOVATION WORKS (GUANGZHOU) ARTIFICIAL INTELLIGENCE RESEARCH CO., LTD.) 29 May 2020 (2020-05-29) entire document | 1-17 |
| A | CN 108140131 A (ATOMWISE INC.) 08 June 2018 (2018-06-08) entire document | 1-17 |
| A | WO 2005081158 A2 (NOVARTIS AG. et al.) 01 September 2005 (2005-09-01) entire document | 1-17 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **13 May 2022** | **25 May 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

| International application No. |
| --- |
| **PCT/CN2022/078336** |

| Patent document cited in search report | | | Publication date (day/month/year) | Patent family member(s) | | | Publication date (day/month/year) |
| --- | --- | --- | --- | --- | --- | --- | --- |
| CN | 112201301 | A | 08 January 2021 | None | | | |
| CN | 108205613 | A | 26 June 2018 | None | | | |
| CN | 111209468 | A | 29 May 2020 | None | | | |
| CN | 108140131 | A | 08 June 2018 | ES | 2772687 | T3 | 08 July 2020 |
| | | | | EP | 3680820 | A1 | 15 July 2020 |
| | | | | EP | 3356999 | A1 | 08 August 2018 |
| | | | | WO | 2017062382 | A1 | 13 April 2017 |
| | | | | JP | 2019501433 | A | 17 January 2019 |
| | | | | IN | 201847016341 | A | 11 May 2018 |
| | | | | SG | 11201802759 | A1 | 30 May 2018 |
| WO | 2005081158 | A2 | 01 September 2005 | None | | | |

Form PCT/ISA/210 (patent family annex) (January 2015)

**REFERENCES CITED IN THE DESCRIPTION**

**Patent documents cited in the description**

• CN 202110260343